# EUROPEAN PATENT APPLICATION

(11) **EP 1 247 867 A1**
(43) Date of publication of application: **09.10.2002**
(21) Application number: 01108682.4
(22) Date of filing: 06.04.2001
(51) Int. Cl.: C12N 15/82

(54) **Gene encoding plant resistance protein to the tomato mosaic tobamovirus (ToMV)**

(71) Applicant: Syngenta Participations AG, 4058 Basel (CH)
(72) Inventor: The designation of the inventor has not yet been filed
(74) Representative: Bastian, Werner et al

(57) **Abstract**

The invention relates to a nucleic acid and the encoded plant resistence protein which upon tabamovirus infection interacts with the 30K tobamovirus movement protein to protect the plant against the spread of the infection. Simultaneous expression of the resistance protein and a 30K movement protein, can be used in a general method of protecting plants from the sprad of a pathogen infection.

## Description

The invention relates to a nucleic acid and the enoded plant resistance protein which upon tobamovirus infection interacts with the 30K tobamovirus movement protein to protect the plant against the spread of the infection.

Disease resistance in plants generally requires the recognition, i.e. incompatible interaction of a specific pathogen protein encoded by the avirulence gene (*avr*) with a specific plant protein encoded by the plant resistance gene (*R*). This interaction leads to the induction of one or more defense responses of the plant. Incompatible plant pathogen interactions can give rise to the induction of a hypersensitive response (HR) in plants, which restricts cell death to the infection site and renders the plants resistant.

Many vegetable and ornamental crops suffer from infections with virulent tobamoviruses which are stable, rod-shaped, non-enveloped particles containing positive stranded linear RNA genomes encapsidated by coat protein. Infection usually gives rise to characteristic mosaic symptoms on leaves and finally to necrosis of tissue, thus leading to yield losses and cosmetical damage. Genetic sources of resistances are widely exploited in breeding programs for many commercial crops. In tomato for example three different resistance genes to tomato mosaic tobamovirus (ToMV) have been used in breeding: *Tm-1*, *Tm-2* and *Tm2*^{*2*} (also referred to as *Tm2*^{*a*}). The latter two genes are supposed to be allelic and are located on chromosome 9 of tomato.

Within the context of the present invention reference to a gene is to be understood as reference to a DNA coding sequence associated with regulatory sequences, which allow transcription of the coding sequence into RNA. Examples of regulatory sequences are promoter sequences, 5' and 3' untranslated sequences, introns, and termination sequences.
A promoter is understood to be a DNA sequence initiating transcription of an associated DNA sequence, and may also include elements that act as regulators of gene expression such as activators, enhancers, or repressors.
Expression of a gene refers to its transcription into RNA or its transcription and subsequent translation into protein within a living cell. The gene can either be part of the genomic DNA of the cell or a gene of a pathogen infecting the cell. Whereas genes which are part of the genomic DNA and genes of an infecting virus are expressed by the transcription and translation machinery of the infected cell, genes of infecting bacteria, fungi or nematodes are expressed by the trancription and translation machinery of these pathogens.
The term transformation of cells designates the introduction of nucleic acid into a host cell, particularly the stable integration of a DNA molecule into the genome of said cell.
Any part or piece of a specific nucleotide or amino acid sequence is referred to as a component sequence.

To improve plant disease resistance in general by means of genetic engineering it is the main objective of the present invention to provide a nucleic acid with an open reading frame (ORF) for a plant resistance protein which, when expressed in a plant cell, induces a HR upon pathogen infection.
The nucleic acid provided by the present invention encodes a resistance protein which induces a HR, thereby killing a plant cell simultaneously expressing the resistance protein and a 30K movement protein of an avirulent tobamovirus strain which would naturally enter the cell upon viral infection. Thus, simultaneous expression of the resistance protein and a tobamovirus 30K movement protein in a plant cell kills said cell. A number of tobamoviruses genomic nucleotide sequences including sequences encoding their 30K movement protein are known. A list of selected strains and corresponding Genbank or Swissprot Accession Numbers of their movement protein or gene sequences is given in Table 1.

**Table 1:**

| Tobamovirus strains | |
|---|---|
| **Acession No.** | **Virus strain** |
| AF187045 | Ribgrass mosaic virus from Brassica chinensis |
| AB003936 | Crucifer tobamovirus (strain:wasabi) |
| S48700 | Tobacco mosaic virus |
| AAC02748 | Turnip vein-clearing virus (strain: OSU) |
| Z92909 | Tobacco mosaic virus (K2 strain) |
| AJ243571 | Tobacco mosaic virus (Kazakh strain K1) |
| AJ132845 | Tomato mosaic virus (S-1) |
| 352986 | Tobacco mosaic virus (strain: L) |
| P29799 | Tomato mosaic virus (strain LIIa, Tm-2 breaker) |
| D17458 | Tobacco mosaic virus (Tm-2² breaker) |
| P29800 | Tomato mosaic virus (strain LIIa, Tm-2 breaker) |
| 352986 (+mut)^{a} | Tobacco mosaic virus (strain: Ltb1, Tm-2 breaker) |
| 352986(+mut)^{a} | Tobacco mosaic virus (strain: ToMV-2², Tm-2² breaker) |
| AF155507 | Tobacco mosaic virus (attenuated tomato mosaic virus K) |
| AF042032 | Tomato mosaic virus (strain: ToMV-38) |
| JC1339 | Tobacco mosaic virus |
| P30737 | Tobacco mosaic virus (isolated in Korea) |
| AJ006991 | Tobacco mosaic virus strain B from broad bean |
| AF165190 | Tobacco mosaic virus (China) |
| AF273221 | Tobacco mosaic virus |
| V01409 | Tobacco mosaic virus (variant 2) |
| 350757 | Tobacco mosaic virus (OM strain) |
| P03582 | Tobacco mosaic virus (OM strain) |
| D63809 | Tobacco mosaic virus (strain:Rakkyo) |
| AF042033 | Tobacco mosaic virus-U1(D) |
| U89894 | Odontoglossum ringspot virus |
| Q84123 | Odontoglossum ringspot virus |
| S83257 | Odontoglossum ringspot virus (Cy-1) |
| U34586 | Odontoglossum ringspot virus (strain:Singapore 1) |
| M81413 | Tobamovirus Pepper mild mottlevirus (strain:S) |
| AAA47936 | Tobacco mild green mosaic virus |
| M34236 | Tobacco mild green mosaic virus (strain PV 228) |
| D13438 | Tobacco mosaic virus (strain:Ob) |
| P25034 | Cucumber green mottle mosaic virus (SH strain) |
| AJ243353 | Cucumber green mottle mosaic virus-Y |
| J04322 | Cucumber green mottle mosaic virus (watermelon strain) |
| AB015145 | Cucumber green mottle mosaic virus (strain:Yodo) |
| AB015144 | Cucumber green mottle mosaic virus (strain:C) |
| AF321957 | Cucumber fruit mottle mosaic virus |
| AF165884 | Frangipani mosaic virus |
| J02413 | Tobacco mosaic virus (cowpea strain) |

| | |
|---|---|
| ^{a}: in this accesion only the wt strain is given, in the cited paper the mutations, which are required to obtain the breaker strains are described. | |

In a plant which is not resistant to the virus the 30K movement protein facilitates spread of the virus from cell-to-cell and through the plant (long distance spreading through the plant is facilitated by the coat protein) by altering the size exclusion limit of the plasmodesmata to assist the passage of (+) strand RNA. In tomato plants harbouring one of the resistance genes *Tm-1*, *Tm-2* or *Tm2*^{*2*} a localized infection with virulent tobamovirus strains does not lead to spread of the infection, because an incompatible interaction between the resistance proteins encoded by these genes and the tobamovirus replicase protein (*Tm-1*) or 30K movement protein (*Tm-2* or *Tm-2*^{*2*}, avirulence proteins of tobamoviruses) induces a defensive HR in plants leading to cell death restricted to the infection site. Whereas the *Tm-1* and *Tm-2* mediated resistance has been overcome by several tobamovirus isolates, resistance conferred by the *Tm-2*^{*2*} gene has turned out to be durable, i.e. the resistance has not been overcome in time on a large scale by virulent tobamovirus strains. In addition, the resistance conferred by *Tm-2*^{*2*} has a broad spectrum activity, i.e. the resistance holds against all tobamovirus strains and isolates which are able to infect tomato. The few observed tobamovirus mutants which were able to break the resistance conferred by *Tm-2*^{*2*} (e.g. the strains described under the GenBank Accession No. D17458 and 352986) turned out to be affected in their virulence and could be controlled by removing the infected plants. This is explained by the fact that, in order to be able to overcome the resistance conferred by *Tm-2*^{*2*}, a virus requires specific mutations in the carboxy terminal part of the 30K movement protein which is a key component for the spread of the virus. The amino acid sequence of a specific embodiment of a tomato mosaic tobamovirus 30K movement protein which cannot overcome resistance conferred by either Tm-2 or Tm-2² is given in SEQ ID NO: 5.

A nucleic acid with an open reading frame for a plant resistance protein according to the present invention is characterized by an encoded amino acid sequence comprising a component sequence of at least 50 amino acid residues having 60% or more identity with an aligned component sequence of SEQ ID NO: 1. In particular the protein encoded by the open reading frame can be described by the formula R₁-R₂-R₃, wherein
-- R₁, R₂ and R₃ constitute component sequences consisting of amino acid residues independently selected from the group of the amino acid residues Gly, Ala, Val, Leu, IIe, Phe, Pro, Ser, Thr, Cys, Met, Trp, Tyr, Asn, Gln, Asp, Glu, Lys, Arg, and His,
-- R₁ and R₃ consist independently of 0 to 1500 amino acid residues;
-- R₂ consists of at least 50 amino acid residues; and
-- R₂ is at least 60% identical to an aligned component sequence of SEQ ID NO: 1.
In most cases the total length of the protein will be in the range of 600 to 1000 amino acid residues. In preferred embodiments of the invention the component sequence R₂ consists of at least 100 amino acid residues. Specific examples of component sequence R₂ are component sequences of SEQ ID NO: 1 defined by the following ranges of amino acids:
-- 1-91 (heptad leucine zipper region)
-- 92-483 (NB-ARC region) preferably amino acids 182-281, 260-359, 339-438, 384-483; 154-203, 182-231, 240-289 or 242-291
-- 477-861 (leucine rich repeat region)

Particularly preferred embodiments of the DNA according to the present invention encode a protein having a component sequence defined by amino acids 1-13, 14-20, 21-27, 28-34, 35-41, 42-48, 49-55, 63-69, 70-76, 77-84, or 85-91 (heptad repeat regions) or amino acids 181-195, 250-260, 279-291, 315-325, 340-348, 353-367, 397-402, 405-415 or 479-483 (NB-ARC motifs) of SEQ ID NO: 1. Preferably, the encoded protein comprises at least two, three or more different representatives of said component sequences. A specific example of said embodiment encodes a protein characterized by the amino acid sequence of SEQ ID NO: 1 (Tm-2²).

Dynamic programming algorithms yield different kinds of alignments. In general there exist two approaches towards sequence alignment. Algorithms as proposed by Needleman & Wunsch and by Sellers align the entire length of two sequences providing a global alignment of the sequences. The Smith-Waterman algorithm on the other hand yields local alignments. A local alignment aligns the pair of regions within the sequences that are most similar given the choice of scoring matrix and gap penalties. This allows a database search to focus on the most highly conserved regions of the sequences. It also allows similar domains within sequences to be identified. To speed up alignments using the Smith-Waterman algorithm both BLAST (Basic Local Alignment Search Tool) and FASTA place additional restrictions on the alignments.

Within the context of the present invention alignments are conveniently performed using BLAST, a set of similarity search programs designed to explore all of the available sequence databases regardless of whether the query is protein or DNA. Version BLAST 2.0 (Gapped BLAST) of this search tool has been made publicly available on the internet (currently http://www.ncbi.nlm.nih.gov/BLAST/). It uses a heuristic algorithm which seeks local as opposed to global alignments and is therefore able to detect relationships among sequences which share only isolated regions. The scores assigned in a BLAST search have a well-defined statistical interpretation. Particularly useful within the scope of the present invention are the blastp program allowing for the introduction of gaps in the local sequence alignments and the PSI-BLAST program, both programs comparing an amino acid query sequence against a protein sequence database, as well as a blastp variant program allowing local alignment of two sequences only. Said programs are preferably run with optional parameters set to the default values.

Sequence alignments using BLAST can also take into account whether the substitution of one amino acid for another is likely to conserve the physical and chemical properties necessary to maintain the structure and function of the protein or is more likely to disrupt essential structural and functional features of a protein. Such sequence similarity is quantified in terms of a percentage of "positive" amino acids, as compared to the percentage of identical amino acids and can help assigning a protein to the correct protein family in border-line cases.

Resistance genes that mediate resistance to viruses, bacteria, fungi, and nematodes have been cloned from several plant species. Several groups of resistance proteins can be discriminated. One of these groups is characterized by the presence of a coiled-coil (CC) domain or more specifically a leucine zipper domain, which is a subgroup in the group of the coiled-coil domains, followed by a nucleotide binding (NB-ARC) domain and a leucine-rich repeat (LRR) region. The latter region is supposed to be involved in recognition and binding of the avirulence protein whereas the coiled coil domains are known for their role in homo-and heterodimerization as well as transmission of a signal to the signal transduction chain. Nucleotide binding domains are also believed to take part in signal transduction.

Sequence alignments using such computer programs as mentioned above reveal the presence of a leucine zipper region containing 8-10 heptad repeats (amino acids 1 to 91 in SEQ ID NO: 1). Alignment additionally reveals a nucleotide binding NB-ARC region (spanning amino acid positions 92 to 483 in SEQ ID NO: 1), and a leucin rich repeat region spanning amino acid positions 477 to 861 in SEQ ID NO: 1 with the consensus repeat region containing 15 imperfect repeats.

Specific examples of DNA according to the present invention are described in SEQ ID NO: 2 and SEQ ID NO: 4 (nucleotide sequences) encoding tomato resistance proteins described in SEQ ID NO: 1 and SEQ ID NO: 3. Stretches of SEQ ID NO: 1 having 50 to 500 amino acids length can show between 20 and 50% sequence identity to stretches of known protein sequences after alignment. Overall alignments of SEQ ID NO: 1, however, result in sequence identities lower than 30%. Thus, the present invention defines a class of pathogen resistance proteins which induce a HR in a plant cell simultaneously expressing the resistance protein and a 30K movement protein of a virulent tobamovirus. Members of said class of proteins are characterized by an amino acid sequence comprising a component sequence of at least 50 amino acid residues having 60% or more identity with an aligned component sequence of SEQ ID NO: 1. Preferably the amino acid sequence identity is higher than 75% or even higher than 90%.

DNA encoding tobamovirus resistance proteins according to the present invention can be isolated from plant species such as *Lycopersicon peruvianum* and *Lycopersicon esculentum*. The following general method, can be used, which the person skilled in the art knows to adapt to the specific task. A single stranded fragment of SEQ ID NO: 2 or SEQ ID NO: 4 consisting of at least 15, preferably 20 to 30 or even more than 50 consecutive nucleotides is used as a probe to screen a DNA library for clones hybridizing to said fragment. The factors to be observed for hybridization are described in Sambrook et al, Molecular cloning: A laboratory manual, Cold Spring Harbor Laboratory Press, chapters 9.47-9.57 and 11.45-11.49, 1989. Hybridizing clones are sequenced and DNA of clones comprising a complete coding region encoding a protein characterized by an amino acid sequence comprising a component sequence of at least 50 amino acid residues having 60% or more sequence identity to SEQ ID NO: 1 is purified. Said DNA can then be further processed by a number of routine recombinant DNA techniques such as restriction enzyme digestion, ligation, or polymerase chain reaction analysis.

The disclosure of SEQ ID NO: 2 and SEQ ID NO: 4 enables a person skilled in the art to design oligonucleotides for polymerase chain reactions which attempt to amplify DNA fragments from templates comprising a sequence of nucleotides characterized by any continuous sequence of 15 and preferably 20 to 30 or more basepairs in SEQ ID NO: 2 or SEQ ID NO: 4. Said nucleotides comprise a sequence of nucleotides which represents 15 and preferably 20 to 30 or more basepairs of SEQ ID NO: 2 or SEQ ID NO: 4. Polymerase chain reactions performed using at least one such oligonucleotide and their amplification products constitute another embodiment of the present invention.

A further embodiment of the present invention is a method of protecting plants comprising a nucleic acid according to the present invention from the spread of a pathogen infection by transforming the plant with a nucleic acid encoding a 30K movement protein of an avirulent tobamovirus, wherein either the expression of the tobamovirus 30K movement protein or the expression of the nucleic acid according to the present invention or the expression of both is controlled by a pathogen-inducible promoter.
In a similar method of protecting plants from the spread of a pathogen infection the plant is transformed with the nucleic acid according to the present invention and a nucleic acid encoding a 30K movement protein of a virulent tobamovirus, wherein either the expression of the nucleic acid according to the present invention or the expression of the tobamovirus 30K movement protein or the expression of both is controlled by a pathogen-inducible promoter.
While any gene encoding a 30K movement protein of a virulent tobamovirus strain can be used in this method a preferred movement protein is the tomato mosaic tobamovirus 30K movement protein as defined in SEQ ID NO: 5 and encoded by the open reading frame defined in SEQ ID NO: 6. Only the few tobamovirus 30K movement protein genes known to break tobamovirus resistance conferred by the *Tm-2*^{*2*} gene cannot be used in this method. This is due to specific mutations in the region encoding the carboxy-terminal part of the protein replacing the Serine residue in position 238 by an Arginine residue and the Lysine residue in position 244 by a Glutamine residue (Weber et al. (1993) J. of Virol. 67:6432-38).

Examples of pathogen-inducible promoters are the GST-1 promoter (Hahn et al, Eur. J. Biochem. 226: 619-626, 1994), the HSR203J promoter (Pontier et al, Plant J. 5: 507-521, 1994), the PDF1.2 promoter (Manners et al, Plant Mol. Biol. 38:1071-1080, 1998) and promoters of PR genes such as the PR-1a promoter (Linthorst, Crit. Rev. Plant Sci. 10:123-150, 1991). Further embodiments can be isolated from plant viruses (Hong et al, Virology 220: 119-127, 1996) or plant genomes (Rushton et al, Curr. Opinion in Plant Biol. 1:311-315, 1998). Upon infection with a pathogen, the protein whose expression is controlled by an inducible promoter accumulates to allow for an incompatible interaction between the movement protein and the resistance protein which in turn induces a hypersensitive reaction avoiding spread of the pathogen (de Wit et al, Ann. Rev. Phytopathol. 30: 391-418, 1992). As a consequence the transgenic plants show high levels of resistance against a broad range of plant pathogens, including viruses, bacteria, fungi and nematodes. Viral pathogens include but are not limited to Geminiviruses, Tospoviruses, Cucumoviruses, Potyviruses, Potexviruses, Tobamoviruses, Luteoviruses or Poleroviruses. Bacterial pathogens include but are not limited to *Pseudomonas* spp., *Xanthomonas* spp. *Erwinia* spp. or *Clavibacterspp.* Fungal pathogens include but are not limited to *Botrytis* spp., *Phytophthora* spp., *Oidium* spp., *Leveillula* spp., *Fusarium* spp., *Verticillium* spp., *Pythium* spp., *Peronospora* spp., *Pyrenochaeta* spp., *Alternaria* spp., *Stemphillium* or *Cladosporium* spp. Nematode pathogens include but are not limited to *Meloidogyne* spp.

Said method can be used in almost any plant especially those belonging to the Family *Solanaceae,* tomato, pepper egg-plant, potato, tobacco and preferably in corn, sugarbeet, sunflower, winter oilseed rape, soybean, cotton, wheat, rice, broccoli, cauliflower, cabbage, cucumber, sweet corn, daikon, bean, lettuce, melon, squash or watermelon.

### Examples

### Example 1: Transposon tagging

A two component *Ac*/*Ds* transposon system is used to isolate the *Tm-2*^{*2*} gene of tomato. For this purpose a tomato genotype (line Ds₁₃₋₁₅) with a *Ds*_{*13-15*}-transposon (in the following also referred to as *Ds)* on chromosome 9 and approximately 2 centiMorgan from the *Tm-2*^{*2*} gene is used. This genotype is constructed by transforming a tomato line homozygous for the *Tm-2*^{*2*} gene with the binary vector pJasm13 (Rommens et al. (1993) Plant Mol. Biol. 21:1109-19; Thomas et al. (1994) Mol. Gen. Genet. 242:573-85; Knapp et al. (1994) Mol. Gen. Genet. 243:666-73). This plasmid contains the *Ds* transposon and both the NPTII and HPTII antibiotic resistance genes that allow selection of the transgenic plants. The genetic distance between *Tm-2*^{*2*} and the *Ds*-transposon insertion is determined by crossing tomato genotype *Ds*_{*13-15*} with tomato genotype ATV840, lacking the *Tm-2*^{*2*} resistance gene (homozygous for *tm-2).* The resulting progeny is again crossed with ATV840. From this population 67 plants are analyzed for the presence of *Ds*_{*13-15*} and *Tm-2*^{*2*}*.* In only one plant the linkage was found to be broken, implying that the genetic distance between *Ds*_{*13-15*} and the *Tm-2*^{*2*} gene is in the range of 2 centiMorgan.

Simultaneous expression of the *ToMV MP* gene and the *Tm-2*^{*2*} gene in one cell is lethal to the cells (Weber & Pfitzner (1998) Mol. Plant Microbe Int. 6:498-503). Crossing transgenic plants expressing *ToMV MP* with plants homozygous for *Tm-2*^{*2*} results in seeds, heterozygous for both genes, which germinate but become necrotic and die when the roots are approximately 5 mm in length. This phenomenon serves as the basis for the transposon tagging experiment. Plants homozygous for *Tm-2*^{*2*} and with a closely linked active transposon are crossed with plants homozygous for the *ToMV MP* gene. Upon germination of the resulting seeds all progeny become necrotic and die except in those cases that one of the two genes (the *Tm-2*^{*2*} gene or the *MP gene)* is inactivated. Predominant mutations in the *Tm-2*^{*2*} gene are anticipated because of the tight linkage of the transposon to the *Tm-2*^{*2*} gene.

To obtain an activated transposon it is necessary to introduce a stabilized Activator (*sAc*) in tomato genotype *Ds*_{*13-15*}, containing the *Ds* transposon and the *Tm-2*^{*2*} gene. For this purpose genotype ATV847 (homozygous for *Tm-2*^{*2*}) is crossed with genotype MMSLJ10512 (homozygous for *sAc*; Takken *et al*., Plant Journal 14: 401-411, 1998; see also Table 2). Selfings from the progeny of this cross are selected for homozygousity of both *sAc* and *Tm-2*^{*2*} via PCR. One of the plants designated TmSLJ is subsequently used in a cross with genotype *Ds*_{*13-15*}*.* Progeny of this cross is selected via PCR for the presence of *Ds, sAc* and for homozygousity of Tm-2². Finally, approximately 100 independent plants with the genotype *Ds,*-; *sAc,*-; *Tm-2*^{*2*}*, Tm-2*^{*2*} are selected for carrying out the large scale tagging experiment and are used as males and females in a cross with tomato line ATV840-4352 which is homozygous for *MP.* In practice, females, homozygous from *Tm-2*^{*2*}, are difficult to pollinate, as a result of flower morphology.

**Table 2:**

| Tomato plant lines. | |
|---|---|
| Line | Genotype |
| MoneyMaker-vir | *Tm-2*^{*2*}, *Tm-2*^{*2*} |
| Ds₁₃₋₁₅ (MoneyMaker-vir) ATV840 (Novartis) | *Ds*,*Ds*; *Tm-2*^{*2*}*,Tm-2*^{*2*} *tm-2,tm-2* |
| ATV847 (Novartis) | *Tm-2*^{*2*}*,Tm-2*^{*2*} |
| ATV840-4352 (Novartis) | *MP,MP; tm-2,tm-2* |
| MMSLJ10512 (Takken *et al*., 1998) | *sAc,sAc;* tm-2,tm-2 |
| TmSLJ | *sAc,sAc; Tm-2*^{*2*}*,Tm-2*^{*2*} |
| Stevens | *Sw5,Sw5; tm-2,tm-2* |

### Example 2: Identification of transposon-tagged mutants

Crosses of the approximately 100 independent plants with the genotype *Ds,* -; *sAc,*-; *Tm-2*^{*2*}*,Tm-2*^{*2*} with line ATV840-4352 (homozygous for *MP*) result in approximately 200,000 seeds. About 140,000 seeds are used in a germination assay. The results of these germination experiments are presented in Table 3. Four different phenotypes can be observed: non-germinating seeds, seeds with approximately 5 mm-long necrotic roots, germinating seeds which become necrotic after cotyledon expansion and normal seedlings. From preliminary experiments one would anticipate that 1 seedling out of 1000 seeds should survive. Unexpectedly however, 1 seedling out of 80 seeds survives. A relation between the number of surviving seedlings and the position of the flower cluster used for pollination can be observed. For this reason, it is decided to concentrate on the survivors obtained from seeds from the first (lowest) three pollinated flower clusters.

**Table 3:**

| Germination of Tomato Seeds from the crossing between genotype *Ds,* -; *sAc,* -; *Tm-2*^{*2*}*, Tm-2*^{*2*} and genotype *MP and* their germination phenotype | | | |
|---|---|---|---|
| Stage | Origin (clusters) | number | (%) |
| Sown seeds | 1-6 | 140,000 | (100) |
| Germinated seeds | 1-6 | 119,600 | (85.4) |
| Seeds with necrotic root | 1-6 | 112,000 | (80.0) |
| Non-surviving seedlings | 1-6 | 5,900 | (4.21) |
| Normal seedlings (putative mutants) | 1-6 | 1,700 | (1.21) |
| Seedlings analyzed | 1-3 | 450 | |
| Mutants obtained | 1-3 | 6 | |

A preliminary screen of 60 surviving plants reveals that many loose the RFLP marker tightly linked to the *Tm-2*^{*2*} gene. This indicates that, in addition to transposon insertions in the *Tm-2*^{*2*} gene, deletions occur which remove both the RFLP marker and the *Tm-2*^{*2*} gene. Subsequently, 450 of the 1700 surviving plants are further analyzed. 6 (#58, #65, #68, #107, #108, #144) plants are shown to possess the RFLP marker. These six plants are putative tagged mutants.

### Example 3: Analysis of the putative tagged mutants

The 6 putative mutants are analyzed in more detail. Each of them contains the *Ds* element, the *Tm-2*^{*2*} linked RFLP marker, and the movement protein gene. None except #108 contains a stabilized activator *sAc*. To test whether these 6 mutants are genuine mutants in the *Tm-2*^{*2*} gene, cuttings of the plants are inoculated with ToMV. Three weeks after inoculation the plants are visually inspected for viral symptoms. In addition, leaf extracts are prepared and re-inoculated on *Nicotiana glutinosa* (ToMV induces local lesions on this host). Five of the putative mutants are susceptible to ToMV in both tests. One putative mutant (#58) remains symptomless and does not accumulate detectable levels of ToMV. Thus, five mutants completely fulfill the criteria for having a transposon insertion in the *Tm-2*^{*2*} gene which knocks out its disease resistance function.

In the surviving plants jumping of the *Ds*-transposon can be observed using an *HPTII* probe specific for the *HPT*-gene in the Ds-transposon (Takken *et al.* 1998 supra). The number of transposons in plants varies from 2 (genotypes Ds₁₃₋₁₅ and TmSLJ and mutants #65, #68, #58, #107, #108) to more then 4 in mutant plant #144. In all the surviving plants jumping of the *Ds* transposon can be demonstrated and two independent insertion events are observed, insertion events like in #68 for plants #58, #65, #68, #107, #108, and the insertion event of plant #144.

### Example 4: DNA isolation, cloning and sequence analysis

The DNA sequence of the regions flanking the transposed *Ds* element in the mutant plants #58, #65, #68, #107, #108, and #144 are rescued according to Rommens *et al.* 1992 supra). After restriction analysis and sequencing four groups of rescued plasmids are are distinguished. With both SacI and BamHI which rescue opposite flanking regions of the transposon, two groups are identified, a B68- and a B144-group, an S68- and an S144-group. BamHI and SacI rescues of plants #58, #65, #68, #107 and #108 result in essentially the same plasmids belonging to the B68 or S68-group. Only plant #144 result in different plasmids. The B68-group plasmids yield 7.5 kb of plant DNA and the S68-group plasmids 2.3 kb of plant DNA. Occasionally extra BamH/BamHI- or extra SacI/SacI-fragments are present which most likely originate from the tomato chromosomal DNA. They do not show any similarity to known resistance genes and are not further analyzed.

Tomato DNA is isolated according to Van Der Beel *et al.* (1992). Southern hybridizations and labeling of the probes is performed according to standard procedures (Church and Gilbert, 1984; Sambrook *et al.,* 1989). Genomic DNA is digested either with BamHI or Sacl. BamHl cuts in the *Ds* element but does not interfere with the characteristics allowing independent maintenance of the rescued plasmids in *Eschericia coli*, SacI cuts just before the left border of the *Ds* element and allows isolation of the flanking DNA opposite to the flanking DNA rescued by BamHI cutting. The digestion products are circularized with T4 ligase and subsequently transformed into Epicurian Coli® XL-10 Gold Ultracompetent Cells (Stratagene®) following the instructions of the supplier's manual.

Sequences are analyzed using ClustaIW (Thompson *et al*., 1994), Clone Manager (Scientific & Educational Software) and BLAST (Altschul *et al*., 1990) software. Sequencing of the plasmids, representing the B68 and the S68 group, yield the 5'-end and the 3'-end sequence of the tagged putative *Tm-2*^{*2*} gene. In the 7.5 kb 5'-end an ORF of 1254 bp is found which is closed by a stop-codon. However, this stop-codon originated from the *Ds*-transposon, suggesting that only the 5'-end part of the gene is rescued. Sequencing of the S68-plasmid reveals that this plasmid contains a stretch of plant DNA coding for a continuous polypeptide of 445 amino acids. An 8-bp sequence at the start of this stretch is identical to an 8-bp sequence at the end of the coding region of the B68-plasmids. This is a typical footprint for an Ac-type transposon confirming that this stretch belongs to the N-terminal part of the coding sequence detected in the B68-plasmids. Moreover, the reading frames from both parts of plant DNA are in frame. Thus, the S68-plasmid contains 1340 bps of the ORF which encode the C-terminal half of the *Tm-2*^{*2*} protein and 998 bps of the terminator region.

Restriction analysis of the S68 and S144 group suggest a relation between the two plasmids and can be further studied by Southern blot analysis. As probes useful in these studies are generated from a HindIII/HindIII, a HindIII/BamHI or a HindIII/NsiI restriction fragment of the *Tm-2*^{*2*} gene and are designated HH, HB and a HN. The probes are upstream (HH-probe) or downstream (HB-probe) of the position of the transposon in the 68-group of mutants, or downstream of the position of the transposon in the 144-group of mutants (HN-probe). Hybridizations are performed under stringent conditions, i.e. in 7% (^{w}/ᵥ) SDS, 0.5M Sodiumphosphate buffer pH 7.2, 1 mM Na₃EDTA and 1% (^{w}/ᵥ) BSA, at 60°C. After a short rinse in 2x SSC membranes are washed for 10 min in 2x SSC followed by 5 min in 0.1 % (^{w}/ᵥ) SDS and 0.1x SSC. Southern blot analysis using the HB-probe, derived from plasmid pS68, and the HN-probe, derived from plasmid pS144, reveal the presence of the HN-probe sequence in both the S68 and the S144 group, whereas the HB-probe hybridizes only with the S68-group. Finally a partial sequence-analysis of a BamHI/HindIII fragment of the S144-group reveals that in these plants the transposon is inserted into the resistance gene 805 bp downstream of the location of the insertion site in the S68-group and in the rescue of 1.5 kb of plant DNA. Analysis and sequencing of the B144-group of plasmids reveals that the plant DNA rescued in these plasmids has no obvious relation with the DNA rescued with the B68-group and no relation with other resistance genes.

Analysis of the genotypes ATV840, ATV847, Stevens, and Tm-2 with both the HH-probe and the HN-probe reveal the presence of a single tm-2 or Tm-2 or *Tm-2*^{*2*} gene. Additionally, shifts of hybridizing bands due to transposon insertion are in accordance with the restriction sites present in the approximately 10 kb of rescued Plant DNA with and without transposon insertion.

From mutant plant #144 no BamHI-plasmid with the 5'-end of the putative *Tm-2*^{*2*} gene can be obtained. It is assumed that in mutant plant #144 a major rearranging event has taken place resulting in the deletion of at least the N-terminal half of the ORF. However, plant #144 is still positive for the *Tm-2*^{*2*} RFLP marker.

### Example 5: Analysis of the Tm-2² ORF

The sequences of plasmids pB68 and pS68 allowed the reconstruction of a continuous stretch of 9.8 kb of plant DNA. In this stretch of DNA three ORFs longer then 300 bps are present. They have a length of 324, 456 and 2586 bps respectively. Translation of the longest ORF results in a protein of 861 amino acids with a calculated MW of 98.8 kD and a pl of 8.3. This protein contains all the features that make it a member of the coiled coil (CC)-Nucleotide binding region (NB-ARC)-leucine rich repeat (LRR)-class of resistance proteins (Fig.6). In the first 91 N-terminal amino acids we could recognize 8-10 putative heptad leucine zipper motifs. These motifs are characterized by the motif α-X₂-α-Xₛ, in which α stands for the amino acids I, L, M and V. A NB-ARC domain could be recognized in the amino acid stretch between amino acids 91-483. AII NB-ARC motifs were present (Van der Biezen *et al*., 1998). Using the LRR-consensus X₂-β-X-β-X₄ (β represents I, L, M, V, F, and Y) 15 imperfect leucine rich repeats could be found in the polypeptide stretch ranging from 477-861. According to these assignments the NB-ARC domain and the LRR-domain partially overlap, i.e.motif 5 of the NB-ARC domain was locate in the first LRR.

Sequence alignment of the obtained protein sequence with homologous protein sequences reveals that different alleles of the *Peronospora parasitica* resistance gene RPP13 from *Arabidopsis thaliana* (Bittner-Eddy *et al*., 2000) are its closest homologues. Using the clustaIX-program the Tm-2² protein is aligned with several resistance proteins. The protein shows the highest identity with CC-(NB-ARC)-LRR proteins from *A.thaliana* (highest shared identity of 25% with RPP13). Homology with other resistance proteins from *L.esculentum* such as Mi-1.2, I2, Prf and Sw-5b is considerably less, i.e. 10-14% identical residues (Meyers et al. (1999) The Plant Journal 20:317-332).

### Example 6: Cloning of other alleles using PCR

The demonstration that the *Tm-2*^{*2*} locus is only present as a single copy allowed us to isolate related alleles using *Tm-2*^{*2*}-specific probes. Using specific primers *Tm-2*^{*2*}-like genes (see Table 4) are isolated from plants with a *tm-2* and *Tm-2*^{*2*} genotype (see Table 1).

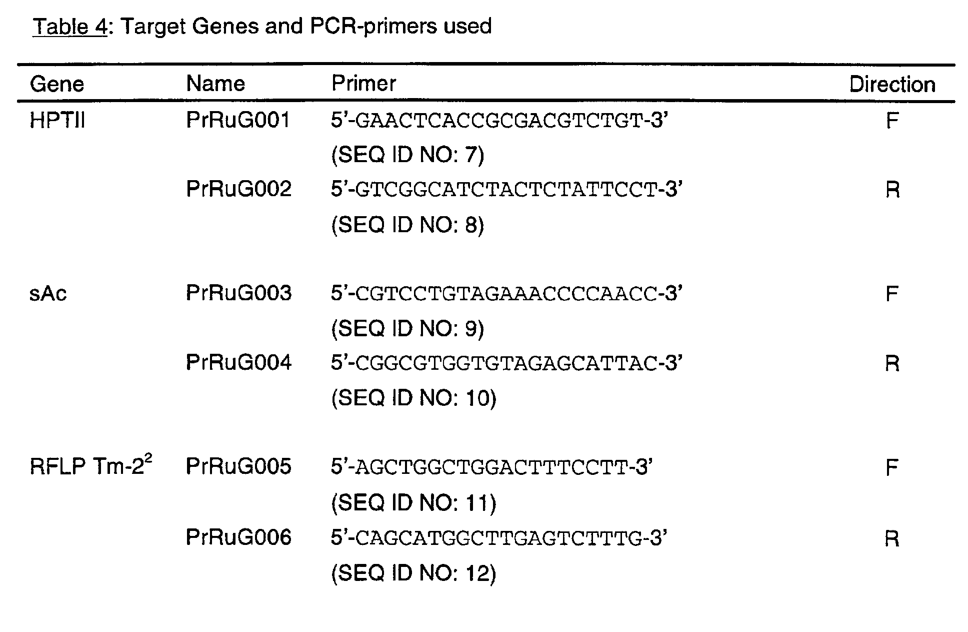

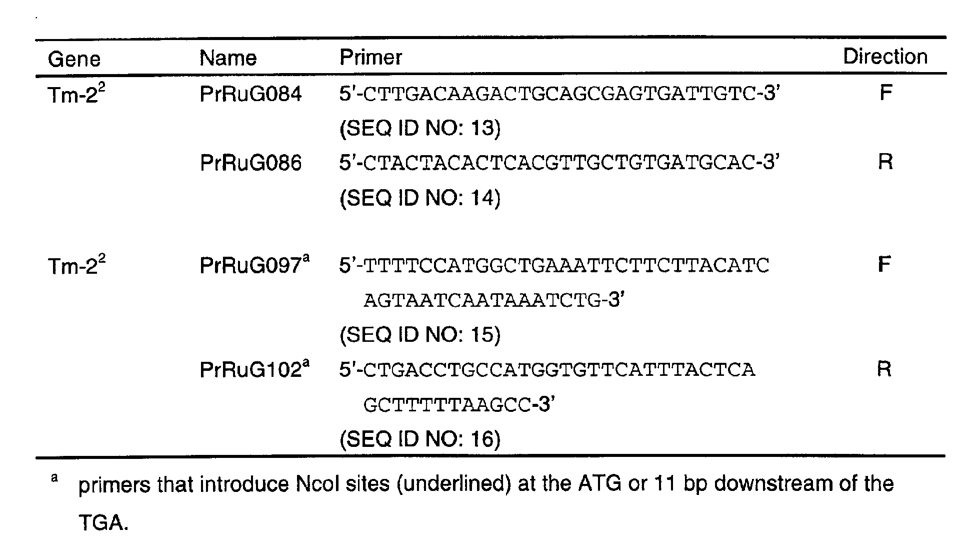

Analysis and comparison of the sequences of *tm-2* and *Tm-2*^{*2*} reveals that all alleles code for a complete and similar type of resistance protein. On the DNA-level no differences between *Tm-2*^{*2*} from Tomato genotype MoneyMaker-vir and *Tm-2*^{*2*} from tomato line ATV847 are observed. This holds true for the *tm-2* alleles from either line Stevens or line ATV840. The nucleotide sequence of the open reading frame found in *tm-2* is given in SEQ ID NO: 4 and the amino acid sequence of the encoded protein in SEQ ID NO: 3. We conclude that both *Tm-2*^{*2*} alleles and both *tm-2* alleles have a common origin and are only separated recently. The difference between *Tm-2*^{*2*} and *tm-2,* however, is considerable. On the DNA level 64 differences (2.3%) are observed, which result in 38 differences (4.4% ) on the protein level (Table 5). Most of the differences are in the C-terminal region of the proteins particularly the LRR-region (compare Table 6).

**Table 5**

| Amino acid differences between the Tm-2² and tm-2 proteins. Amino acid residues are given in the single lettercode. The additional number in the Tm-2² column represents the residues position in the protein (SEQ ID NO: 1) | | | |
|---|---|---|---|
| Tm-2² | tm-2 | Tm-2² | tm-2 |
| Q90 | R | S723 | F |
| R100 | T | K731 | N |
| S221 | G | H737 | K |
| M238 | I | A739 | V |
| V348 | A | N746 | D |
| G413 | S | M749 | I |
| A544 | T | L760 | I |
| Y555 | C | A766 | V |
| R592 | K | Y767 | C |
| P624 | L | S769 | M |
| M704 | I | R772 | S |
| 1707 | T | Y773 | C |
| F708 | C | 1774 | L |
| S709 | R | F781 | L |
| L712 | P | F790 | V |
| E716 | K | D800 | A |
| S721 | R | R849 | G |

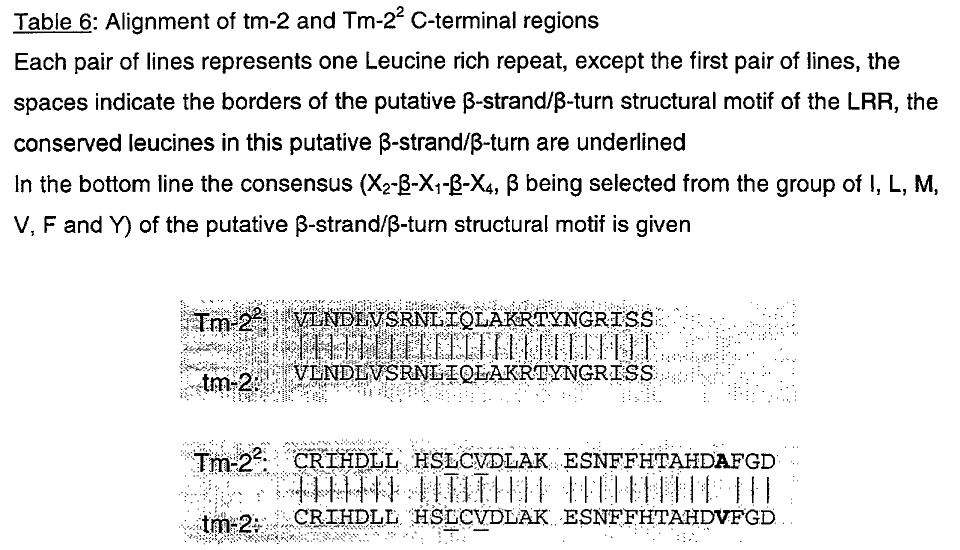

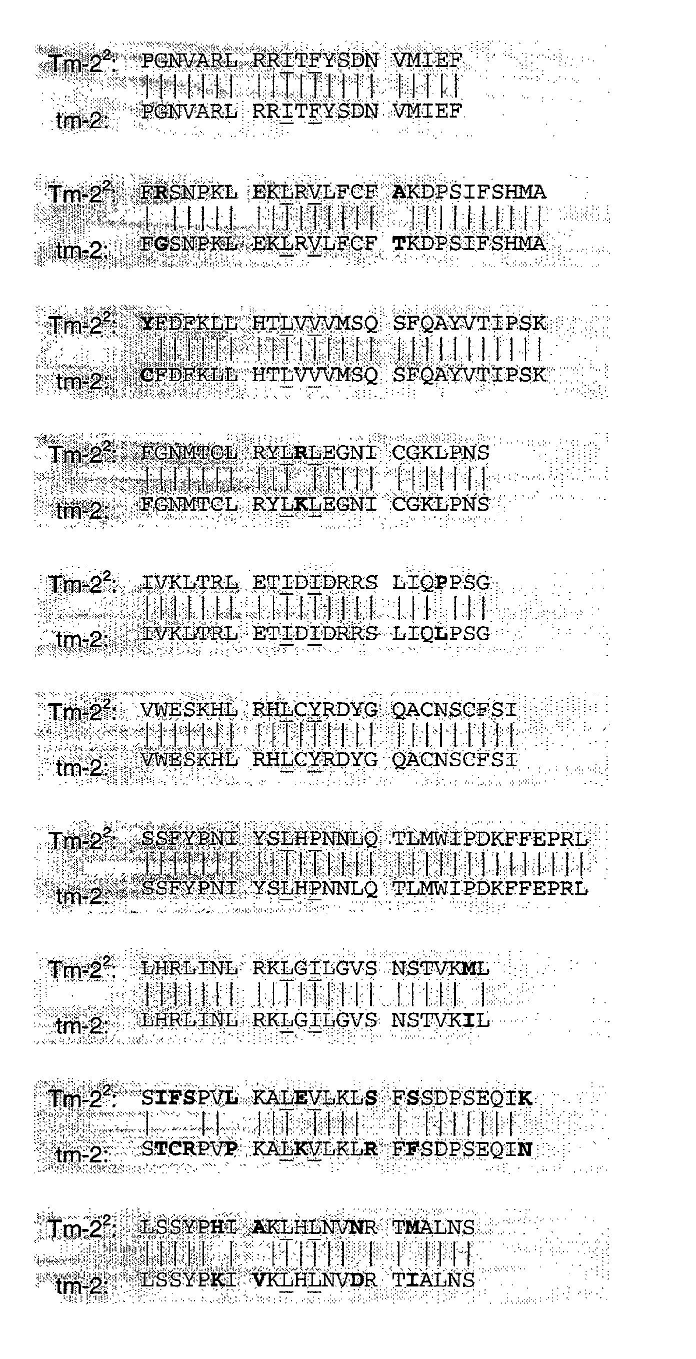

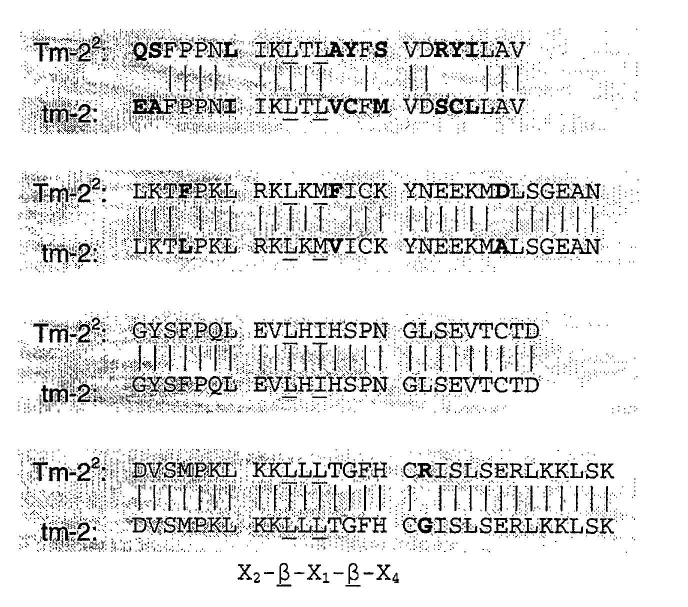

### Example 7: Functional complementation in tomato

Two types of binary vectors are constructed to transfer Tm-2² resistance to non-resistant ATV840 plants with the tm-2 genotype.

### A binary vector with the Tm-2²gene under the control of its own promoter and terminsequences (the original gene)

A fragment carrying the 3'-end of the *Tm-2*^{*2*} gene is excised from pS65 by digesting with Xhol and SacI and, subsequently, cloned into pBluescript (Stratagene) resulting in the plasmid pBlueCterm. Both pB65 and pBlueCterm are digested with Xhol and the Xhol fragment from pB65 carrying the 5'- end of *Tm-2*^{*2*} gene is ligated into pBlueCterm. The relative orientation of the 5'end and the 3'-end is checked by PCR and digestion analysis. The resulting plasmid is named pTm22:Ds and still comprises sequences originating from the Ds-transposon in the *Tm-2*^{*2*} gene. Using primers PrRuG84 and PrRug86 (see Table 4 above) a PCR product is amplified from genomic DNA of the tomato line Ds13₁₃₋₁₅. The PCR product and pTm22:Ds are digested with Aatll and Nhel and the PCR-fragment is cloned into pTm22:Ds resulting in plasmid pTM7. In this plasmid the complete and intact Tm-2² ORF with 750 bp of the *Tm-2*^{*2*} promoter and 1000 bp of the *Tm-2*^{*2*} terminator are present.

Plasmid pTM7 is digested with SacI and Xhol and the *Tm-2*^{*2*} gene is cloned into pZO1560, a pBluescript derivative in which the original multicloning site is replaced by the AGLINK multicloning site (SEQ ID NO: 17) using its SacI and Sall-sites. The resulting plasmid pTM9 is digested with Pad and AscI and the *Tm-2*^{*2*} gene is cloned into the binary vector pVictorHiNK (SEQ ID NO: 5 of WO 00/68374) resulting in plasmid pTM35.

### A binary vector with the Tm-2² gene under the control of the 35S CaMV promoter and the NOS-terminator (the chimaeric gene)

Using primer PrRuG97 and PrRuG102 (see Table 4 above) a PCR product containing the complete ORF of *Tm-2*^{*2*} with an introduced Ncol site at the ATG and an introduced Ncol site 11 bp downstream of the TGA, is amplified from genomic DNA of tomato line ATV847. The PCR-product is digested with Ncol and this fragment is introduced into the Ncol site of pZU-C (see WO 95/09920). The orientation of the ORF relative to the promoter and terminator is checked by digestion and the plasmid named pTM40. pTM40 is digested with BamHI and Xbal and the chimaeric *Tm-2*^{*2*} gene is cloned into the binary vector pVictorHiNK, resulting in plasmid pTM42.

### Plant transformation

The plasmids pTM35 and pTM42 are introduced into *Agrobacterium tumefaciens* strain LBA4404 by triparental matings using pRK2013 as a helper plasmid (Horsch *et al*., Science 227: 1229-1231,1985). Subsequently the purified transconjugants are checked for carrying unaltered gene constructs and used to transform tomato line ATV840 leaf explants essentially as described by Horsch *et al.* (1985) with minor modifications. Instead of leaf explants hypocotyl explants are used. The explants are dipped in an *Agrobacterium tumefaciens* suspension and co-cultivated for 48h on co-cultivation medium consisting of Murashige and Skoog salts and vitamins (Duchefa, The Netherlands), 15 g/liter sucrose, 10 g/liter plant agar, supplemented with 0.2 µg/ml 2.4-D and 0.1 µg/ml kinetine. The explants are then cleared from *Agrobacterium tumefaciens* and transferred to selection medium, which is co-cultivation medium supplemented with 10 g/liter glucose, 0.1 µg/ml IAA, 1.0 µg/ml zeatine, 100 µg/ml kanamycin and 250 µg/ml carbenicillin. Subsequently, the developed shoots are transferred to rooting medium (co-cultivation medium supplemented with 30 g/liter sucrose, 8 g/liter plant agar, 100 µg/ml kanamycin and 250 µg/ml carbenicillin). Of the Kanamycin-resistant plantlets 20 plantlets of both the transformations with pTM35 and pTM42 are transferred to soil and grown in the greenhouse under standard greenhouse conditions.

### Virus resistance assays

After 30 days the independent transgenic plants of tomato line ATV840 are inoculated with leave homogenates of *Nicotiana tobacum* plants infected with a dutch greenhouse isolate of ToMV, which is diluted 1:10 in 10 mM Sodium phosphate buffer, pH 7.0 containing 1% Na₂SO₃. Untransformed plants, as well as plants transformed with an empty binary vector are used as controls for virus inoculations. The plants are all inoculated twice in a four day interval to rule out random escape of inoculation. Virus symptoms are monitored on a daily basis for the duration of the experiment (21 days). Transgenic plants remaining symptomless and plants affected by ToMV are checked for the presence of virus in leaves higher than the inoculated leaf by means of inoculating *Nicotiana glutinosa* with leaf homogenates obtained from the tomato plants. From the 20 independent kanamycin resistant tomato transformants with the original *Tm-2*^{*2*} gene 13 plants resistant against ToMV are obtained. From the 20 independent kanamycin resistant tomato transformants with the chimaeric *Tm-2*^{*2*} gene 15 plants resistant against ToMV are obtained. Resistance means that no ToMV symptomes are observed on these plants after inoculation with virus and no virus is obtained from these plants as demonstrated by inoculating *Nicotiana glutinosa* with a leave homogenate of said these plants.

## Claims

1. A nucleic acid comprising an open reading frame encoding a plant resistance protein, wherein simultaneous expression of said resistance protein and a tobamovirus 30K movement protein in a plant cell kills said cell.

2. The nucleic acid of claim 1, wherein the plant resistance protein and the tobamovirus 30K movement protein interact to induce a defense or hypersensitive response.

3. The nucleic acid of claim 1, wherein the tobamovirus 30K movement protein is a tomato mosaic tobamovirus 30K movement protein.

4. The nucleic acid of claim 3, wherein the movement protein has the amino acid sequence of SEQ ID NO: 1.

5. The nucleic acid of claim 1, wherein the plant resistance protein contains a coiled coil, a nucleotide binding and a leucine rich repeat region.

6. The nucleic acid of claim 1, wherein the plant resistance protein is **characterized by** an amino acid sequence comprising a component sequence of at least 50 amino acid residues having 60% or more identity with an aligned component sequence of SEQ ID NO: 1.

7. The nucleic acid of claim 1 encoding a protein having the formula R₁-R₂-R₃, wherein
-- R₁, R₂ and R₃ constitute component sequences consisting of amino acid residues independently selected from the group of the amino acid residues Gly, Ala, Val, Leu, Ile, Phe, Pro, Ser, Thr, Cys, Met, Trp, Tyr, Asn, Gln, Asp, Glu, Lys, Arg, and His,
-- R₁ and R₃ consist independently of 0 to 1500 amino acid residues;
-- R₂ consists of at least 50 amino acid residues; and
-- R₂ is at least 60% identical to an aligned component sequence of SEQ ID NO: 1.

8. The nucleic acid of claim 1 comprising an open reading frame encoding a protein having a component sequence defined by amino acids 182-281, 260-359, 339-438, or 384-483; or a component sequence defined by amino acids 154-203, 182-231, 240-289 or 242-291 of SEQ ID NO: 1.

9. The nucleic acid of claim 1 having the nucleotide sequence of SEQ ID NO: 2 or SEQ ID NO: 4.

10. The protein encoded by the open reading frame of any one of claims 1 to 9.

11. A method of producing DNA according to claim 1, comprising
- screening a DNA library for clones which are capable of hybridizing to a fragment of DNA defined by SEQ ID NO: 2 or SEQ ID NO: 4, wherein said fragment has a length of at least 15 nucleotides;
- sequencing hybridizing clones;
- purifying vector DNA of clones comprising an open reading frame encoding a protein **characterized by** an amino acid sequence comprising a component sequence of at least 50 amino acid residues having 60% or more sequence identity to SEQ ID NO: 1; and
- optionally further processing the purified DNA.

12. A polymerase chain reaction wherein at least one primer oligonucleotide comprises a sequence of nucleotides which represents 15 or more basepairs of SEQ ID NO: 2 or SEQ ID NO: 4.

13. A method of protecting plants comprising a nucleic acid according to claim 1 from the spread of a pathogen infection comprising transforming the plant with a nucleic acid encoding a tobamovirus 30K movement protein, wherein either the expression of the tobamovirus 30K movement protein or the expression of the nucleic acid according to claim 1 or the expression of both is controlled by a pathogen-inducible promoter.

14. A method of protecting plants from the spread of a pathogen infection comprising transforming the plant with the nucleic acid of claim 1 and a nucleic acid encoding a tobamovirus 30K movement protein, wherein either the expression of the nucleic acid according to claim 1, or the expression of the tobamovirus 30K movement protein or the expression of both is controlled by a pathogen-inducible promoter.

15. The method of claims 13 and 15, wherein the tobamovirus 30K movement protein is a tomato mosaic tobamovirus 30K movement protein and the plant resistance protein is **characterized by** an amino acid sequence comprising a component sequence of at least 50 amino acid residues having 60% or more identity with an aligned component sequence of SEQ ID NO: 1.


SEQUENCE LISTING

<110> Syngenta Participations AG

<120> Organic compounds

<130> S-60005/P1

<140>
<141>

<160> 17

<170> PatentIn Ver. 2.1

<210> 1
<211> 861
<212> PRT
<213> Lycopersicon esculentum

<400> 1
Met Ala Glu Ile Leu Leu Thr Ser Val Ile Asn Lys Ser Val Glu Ile
  1               5                  10                  15

Ala Gly Asn Leu Leu Ile Gln Glu Gly Lys Arg Leu Tyr Trp Leu Lys
             20                  25                  30

Glu Asp Ile Asp Trp Leu Gln Arg Glu Met Arg His Ile Arg Ser Tyr
         35                  40                  45

Val Asp Asn Ala Lys Ala Lys Glu Ala Gly Gly Asp Ser Arg Val Lys
     50                  55                  60

Asn Leu Leu Lys Asp Ile Gln Glu Leu Ala Gly Asp Val Glu Asp Leu
 65                  70                  75                  80

Leu Asp Asp Phe Leu Pro Lys Ile Gln Gln Ser Asn Lys Phe Asn Tyr
                 85                  90                  95

Cys Leu Lys Arg Ser Ser Phe Ala Asp Glu Phe Ala Met Glu Ile Glu
            100                 105                 110

Lys Ile Lys Arg Arg Val Val Asp Ile Asp Arg Ile Arg Lys Thr Tyr
        115                 120                 125

Asn Ile Ile Asp Thr Asp Asn Asn Asn Asp Asp Cys Val Leu Leu Asp
    130                 135                 140

Arg Arg Arg Leu Phe Leu His Ala Asp Glu Thr Glu Ile Ile Gly Leu
145                 150                 155                 160

Asp Asp Asp Phe Asn Met Leu Gln Ala Lys Leu Leu Asn Gln Asp Leu
                165                 170                 175

His Tyr Gly Val Val Ser Ile Val Gly Met Pro Gly Leu Gly Lys Thr

            180                 185                 190

Thr Leu Ala Lys Lys Leu Tyr Arg Leu Ile Arg Asp Gln Phe Glu Cys
        195                 200                 205

Ser Gly Leu Val Tyr Val Ser Gln Gln Pro Arg Ala Ser Glu Ile Leu
    210                 215                 220

Leu Asp Ile Ala Lys Gln Ile Gly Leu Thr Glu Gln Lys Met Lys Glu
225                 230                 235                 240

Asn Leu Glu Asp Asn Leu Arg Ser Leu Leu Lys Ile Lys Arg Tyr Val
                245                 250                 255

Ile Leu Leu Asp Asp Ile Trp Asp Val Glu Ile Trp Asp Asp Leu Lys
            260                 265                 270

Leu Val Leu Pro Glu Cys Asp Ser Lys Val Gly Ser Arg Met Ile Ile
        275                 280                 285

Thr Ser Arg Asn Ser Asn Val Gly Arg Tyr Ile Gly Gly Glu Ser Ser
    290                 295                 300

Leu His Ala Leu Gln Pro Leu Glu Ser Glu Lys Ser Phe Glu Leu Phe
305                 310                 315                 320

Thr Lys Lys Ile Phe Asn Phe Asp Asp Asn Asn Ser Trp Ala Asn Ala
                325                 330                 335

Ser Pro Asp Leu Val Asn Ile Gly Arg Asn Ile Val Gly Arg Cys Gly
            340                 345                 350

Gly Ile Pro Leu Ala Ile Val Val Thr Ala Gly Met Leu Arg Ala Arg
        355                 360                 365

Glu Arg Thr Glu His Ala Trp Asn Arg Val Leu Glu Ser Met Gly His
    370                 375                 380

Lys Val Gln Asp Gly Cys Ala Lys Val Leu Ala Leu Ser Tyr Asn Asp
385                 390                 395                 400

Leu Pro Ile Ala Ser Arg Pro Cys Phe Leu Tyr Phe Gly Leu Tyr Pro
                405                 410                 415

Glu Asp His Glu Ile Arg Ala Phe Asp Leu Ile Asn Met Trp Ile Ala
            420                 425                 430

Glu Lys Phe Ile Val Val Asn Ser Gly Asn Arg Arg Glu Ala Glu Asp
        435                 440                 445

Leu Ala Glu Asp Val Leu Asn Asp Leu Val Ser Arg Asn Leu Ile Gln
    450                 455                 460

Leu Ala Lys Arg Thr Tyr Asn Gly Arg Ile Ser Ser Cys Arg Ile His
465                 470                 475                 480

Asp Leu Leu His Ser Leu Cys Val Asp Leu Ala Lys Glu Ser Asn Phe
                485                 490                 495

Phe His Thr Ala His Asp Ala Phe Gly Asp Pro Gly Asn Val Ala Arg
            500                 505                 510

Leu Arg Arg Ile Thr Phe Tyr Ser Asp Asn Val Met Ile Glu Phe Phe
        515                 520                 525

Arg Ser Asn Pro Lys Leu Glu Lys Leu Arg Val Leu Phe Cys Phe Ala
    530                 535                 540

Lys Asp Pro Ser Ile Phe Ser His Met Ala Tyr Phe Asp Phe Lys Leu
545                 550                 555                 560

Leu His Thr Leu Val Val Val Met Ser Gln Ser Phe Gln Ala Tyr Val
                565                 570                 575

Thr Ile Pro Ser Lys Phe Gly Asn Met Thr Cys Leu Arg Tyr Leu Arg
            580                 585                 590

Leu Glu Gly Asn Ile Cys Gly Lys Leu Pro Asn Ser Ile Val Lys Leu
        595                 600                 605

Thr Arg Leu Glu Thr Ile Asp Ile Asp Arg Arg Ser Leu Ile Gln Pro
    610                 615                 620

Pro Ser Gly Val Trp Glu Ser Lys His Leu Arg His Leu Cys Tyr Arg
625                 630                 635                 640

Asp Tyr Gly Gln Ala Cys Asn Ser Cys Phe Ser Ile Ser Ser Phe Tyr
                645                 650                 655

Pro Asn Ile Tyr Ser Leu His Pro Asn Asn Leu Gln Thr Leu Met Trp
            660                 665                 670

Ile Pro Asp Lys Phe Phe Glu Pro Arg Leu Leu His Arg Leu Ile Asn
        675                 680                 685

Leu Arg Lys Leu Gly Ile Leu Gly Val Ser Asn Ser Thr Val Lys Met
    690                 695                 700

Leu Ser Ile Phe Ser Pro Val Leu Lys Ala Leu Glu Val Leu Lys Leu
705                 710                 715                 720

Ser Phe Ser Ser Asp Pro Ser Glu Gln Ile Lys Leu Ser Ser Tyr Pro
                725                 730                 735

His Ile Ala Lys Leu His Leu Asn Val Asn Arg Thr Met Ala Leu Asn
            740                 745                 750

Ser Gln Ser Phe Pro Pro Asn Leu Ile Lys Leu Thr Leu Ala Tyr Phe
        755                 760                 765

Ser Val Asp Arg Tyr Ile Leu Ala Val Leu Lys Thr Phe Pro Lys Leu
    770                 775                 780

Arg Lys Leu Lys Met Phe Ile Cys Lys Tyr Asn Glu Glu Lys Met Asp
785                 790                 795                 800

Leu Ser Gly Glu Ala Asn Gly Tyr Ser Phe Pro Gln Leu Glu Val Leu
                805                 810                 815

His Ile His Ser Pro Asn Gly Leu Ser Glu Val Thr Cys Thr Asp Asp
            820                 825                 830

Val Ser Met Pro Lys Leu Lys Lys Leu Leu Leu Thr Gly Phe His Cys
        835                 840                 845

Arg Ile Ser Leu Ser Glu Arg Leu Lys Lys Leu Ser Lys
    850                 855                 860

<210> 2
<211> 2586
<212> DNA
<213> Lycopersicon esculentum

<400> 2
atggctgaaa ttcttcttac atcagtaatc aataaatctg tagaaatagc tggaaattta 60
ctgattcaag aaggaaagcg tttatattgg ttgaaagagg atatcgattg gctccagaga 120
gaaatgagac acattcgatc ttatgttgac aacgcaaagg ccaaggaagc tggaggtgat 180
tcaagggtca aaaacttatt gaaagatatt caagaattgg caggtgatgt ggaggatctc 240
ttagatgact tccttccaaa aattcaacaa tccaataagt tcaattattg ccttaagagg 300
agttcttttg cagatgagtt tgctatggag attgagaaga taaagagaag ggttgttgac 360
attgaccgaa taaggaaaac ttacaacatc atagatacag ataacaataa tgatgattgt 420
gttctgctgg atcggagaag attattccta catgctgatg aaacagagat catcggtttg 480
gatgatgact tcaatatgct acaagccaaa ttacttaatc aagatttgca ttatggagtt 540
gtttccatag ttggcatgcc cggtctgggg aaaacaactc ttgccaagaa actttatagg 600
ctcattcgtg atcaatttga gtgttctgga ctggtctacg tttcacaaca gccaagagcg 660
agtgaaatct tacttgacat tgccaaacaa attggactga cggaacagaa aatgaaggaa 720
aatttggagg acaacctgcg atcactcttg aaaataaaaa ggtatgttat cctcctagat 780
gacatttggg atgtggaaat ttgggatgat ctgaaacttg tccttcctga atgtgattca 840
aaagtcggca gtagaatgat aatcacgtct cgaaatagta atgtaggcag atacatagga 900
ggggaatcct ccctccatgc attgcaaccc ctagaatccg agaaaagctt tgaactcttt 960
accaagaaaa tctttaattt tgatgataat aatagttggg ccaatgcttc acctgacttg 1020
gtgaatattg gtagaaatat agttgggaga tgtggaggta taccgctagc catagtggtg 1080
actgcaggca tgttaagggc aagagaaaga acagaacatg cgtggaacag agtacttgag 1140
agtatgggcc ataaagttca agatggatgt gctaaggtat tggctctcag ttacaatgat 1200
ttacctattg cctcaaggcc atgtttcttg tactttggcc tttaccccga ggaccatgaa 1260
attcgtgctt ttgatttgat aaatatgtgg attgctgaga agtttatagt agtaaatagt 1320
ggtaataggc gagaggctga ggatttggcg gaggacgtcc taaatgattt ggtttctaga 1380
aacttgattc aacttgccaa aaggacatat aatggaagaa tttcaagttg tcgcatacat 1440
gacttgttac atagtttgtg tgtggacttg gctaaggaaa gtaacttctt tcacaccgcg 1500
catgatgcat ttggtgatcc cggcaatgtt gctaggctcc gaaggattac attctactct 1560
gacaatgtca tgattgagtt cttccgttca aatcctaagc ttgagaagct tcgtgtactt 1620
ttctgtttcg caaaagaccc ttccatattt tctcatatgg cttattttga cttcaaattg 1680
ttgcacacat tggttgtagt catgtctcaa agttttcaag catatgtcac tatcccaagc 1740
aaatttggga acatgacttg cttacgctat ctgagattgg aggggaatat ttgtggaaaa 1800
ctgccaaata gtattgtcaa gctcacacgt ctagagacca tagacattga tcgacgtagc 1860
ctcattcaac ctccttctgg tgtttgggag tctaaacatt tgagacatct ttgttataga 1920

gattatggac aagcatgtaa cagttgcttt tctataagct cattttaccc aaatatttac 1980
tcattgcatc ctaacaatct acaaaccttg atgtggatac ctgataaatt ttttgaaccg 2040
aggttgttgc accgattgat caatttaaga aaactgggta tactgggagt gtccaattct 2100
accgttaaga tgttatcaat atttagccct gtgcttaagg cgctggaggt tctgaagctc 2160
agtttttcca gtgacccgag tgaacaaata aagttgtcat cgtatccaca tattgctaag 2220
ttgcatttga atgttaacag aacaatggcc ttgaactctc aatcatttcc tccaaatctc 2280
atcaagctta ctctagccta ctttagtgta gaccgttata tactggcagt acttaagaca 2340
tttcccaaat taagaaaact taaaatgttc atctgcaagt ataatgaaga aaagatggat 2400
ctctcgggcg aggcaaatgg ttatagcttt ccgcaacttg aagttttgca tattcatagc 2460
ccgaatgggt tgtctgaagt aacgtgcacg gatgatgtca gtatgcccaa attgaaaaag 2520

ctgttactta caggattcca ttgccgaatc agtttatcgg aacggcttaa aaagctgagt 2580
aaatga 2586

<210> 3
<211> 861
<212> PRT
<213> Lycopersicon esculentum

<400> 3
Met Ala Glu Ile Leu Leu Thr Ser Val Ile Asn Lys Ser Val Glu Ile
1               5                   10                  15

Ala Gly Asn Leu Leu Ile Gln Glu Gly Lys Arg Leu Tyr Trp Leu Lys
            20                  25                  30

Glu Asp Ile Asp Trp Leu Gln Arg Glu Met Arg His Ile Arg Ser Tyr
        35                  40                  45

Val Asp Asn Ala Lys Ala Lys Glu Ala Gly Gly Asp Ser Arg Val Lys
    50                  55                  60

Asn Leu Leu Lys Asp Ile Gln Glu Leu Ala Gly Asp Val Glu Asp Leu
65                  70                  75                  80

Leu Asp Asp Phe Leu Pro Lys Ile Gln Arg Ser Asn Lys Phe Asn Tyr
                85                  90                  95

Cys Leu Lys Thr Ser Ser Phe Ala Asp Glu Phe Ala Met Glu Ile Glu
            100                 105                 110

Lys Ile Lys Arg Arg Val Val Asp Ile Asp Arg Ile Arg Lys Thr Tyr
        115                 120                 125

Asn Ile Ile Asp Thr Asp Asn Asn Asn Asp Asp Cys Val Leu Leu Asp
    130                 135                 140

Arg Arg Arg Leu Phe Leu His Ala Asp Glu Thr Glu Ile Ile Gly Leu
145                 150                 155                 160

Asp Asp Asp Phe Asn Met Leu Gln Ala Lys Leu Leu Asn Gln Asp Leu
                165                 170                 175

His Tyr Gly Val Val Ser Ile Val Gly Met Pro Gly Leu Gly Lys Thr
            180                 185                 190

Thr Leu Ala Lys Lys Leu Tyr Arg Leu Ile Arg Asp Gln Phe Glu Cys
        195                 200                 205

Ser Gly Leu Val Tyr Val Ser Gln Gln Pro Arg Ala Gly Glu Ile Leu
    210                 215                 220

Leu Asp Ile Ala Lys Gln Ile Gly Leu Thr Glu Gln Lys Ile Lys Glu
225                 230                 235                 240

Asn Leu Glu Asp Asn Leu Arg Ser Leu Leu Lys Ile Lys Arg Tyr Val
                245                 250                 255

Ile Leu Leu Asp Asp Ile Trp Asp Val Glu Ile Trp Asp Asp Leu Lys
            260                 265                 270

Leu Val Leu Pro Glu Cys Asp Ser Lys Val Gly Ser Arg Met Ile Ile
        275                 280                 285

Thr Ser Arg Asn Ser Asn Val Gly Arg Tyr Ile Gly Gly Glu Ser Ser
    290                 295                 300

Leu His Ala Leu Gln Pro Leu Glu Ser Glu Lys Ser Phe Glu Leu Phe
305                 310                 315                 320

Thr Lys Lys Ile Phe Asn Phe Asp Asp Asn Asn Ser Trp Ala Asn Ala
                325                 330                 335

Ser Pro Asp Leu Val Asn Ile Gly Arg Asn Ile Ala Gly Arg Cys Gly
            340                 345                 350

Gly Ile Pro Leu Ala Ile Val Val Thr Ala Gly Met Leu Arg Ala Arg
        355                 360                 365

Glu Arg Thr Glu His Ala Trp Asn Arg Val Leu Glu Ser Met Gly His
    370                 375                 380

Lys Val Gln Asp Gly Cys Ala Lys Val Leu Ala Leu Ser Tyr Asn Asp
385                 390                 395                 400

Leu Pro Ile Ala Ser Arg Pro Cys Phe Leu Tyr Phe Ser Leu Tyr Pro
                405                 410                 415

Glu Asp His Glu Ile Arg Ala Phe Asp Leu Ile Asn Met Trp Ile Ala
            420                 425                 430

Glu Lys Phe Ile Val Val Asn Ser Gly Asn Arg Arg Glu Ala Glu Asp
        435                 440                 445

Leu Ala Glu Asp Val Leu Asn Asp Leu Val Ser Arg Asn Leu Ile Gln
    450                 455                 460

Leu Ala Lys Arg Thr Tyr Asn Gly Arg Ile Ser Ser Cys Arg Ile His
465                 470                 475                 480

Asp Leu Leu His Ser Leu Cys Val Asp Leu Ala Lys Glu Ser Asn Phe

                485                 490                 495

Phe His Thr Ala His Asp Val Phe Gly Asp Pro Gly Asn Val Ala Arg
            500                 505                 510

Leu Arg Arg Ile Thr Phe Tyr Ser Asp Asn Val Met Ile Glu Phe Phe
        515                 520                 525

Gly Ser Asn Pro Lys Leu Glu Lys Leu Arg Val Leu Phe Cys Phe Thr
    530                 535                 540

Lys Asp Pro Ser Ile Phe Ser His Met Ala Cys Phe Asp Phe Lys Leu
545                 550                 555                 560

Leu His Thr Leu Val Val Val Met Ser Gln Ser Phe Gln Ala Tyr Val
                565                 570                 575

Thr Ile Pro Ser Lys Phe Gly Asn Met Thr Cys Leu Arg Tyr Leu Lys
            580                 585                 590

Leu Glu Gly Asn Ile Cys Gly Lys Leu Pro Asn Ser Ile Val Lys Leu
        595                 600                 605

Thr Arg Leu Glu Thr Ile Asp Ile Asp Arg Arg Ser Leu Ile Gln Leu
    610                 615                 620

Pro Ser Gly Val Trp Glu Ser Lys His Leu Arg His Leu Cys Tyr Arg
625                 630                 635                 640

Asp Tyr Gly Gln Ala Cys Asn Ser Cys Phe Ser Ile Ser Ser Phe Tyr
                645                 650                 655

Pro Asn Ile Tyr Ser Leu His Pro Asn Asn Leu Gln Thr Leu Met Trp
            660                 665                 670

Ile Pro Asp Lys Phe Phe Glu Pro Arg Leu Leu His Arg Leu Ile Asn
        675                 680                 685

Leu Arg Lys Leu Gly Ile Leu Gly Val Ser Asn Ser Thr Val Lys Ile
    690                 695                 700

Leu Ser Thr Cys Arg Pro Val Pro Lys Ala Leu Lys Val Leu Lys Leu
705                 710                 715                 720

Arg Phe Phe Ser Asp Pro Ser Glu Gln Ile Asn Leu Ser Ser Tyr Pro
                725                 730                 735

Lys Ile Val Lys Leu His Leu Asn Val Asp Arg Thr Ile Ala Leu Asn
            740                 745                 750

Ser Glu Ala Phe Pro Pro Asn Ile Ile Lys Leu Thr Leu Val Cys Phe
        755                 760                 765

Met Val Asp Ser Cys Leu Leu Ala Val Leu Lys Thr Leu Pro Lys Leu
    770                 775                 780

Arg Lys Leu Lys Met Val Ile Cys Lys Tyr Asn Glu Glu Lys Met Ala
785                 790                 795                 800

Leu Ser Gly Glu Ala Asn Gly Tyr Ser Phe Pro Gln Leu Glu Val Leu
                805                 810                 815

His Ile His Ser Pro Asn Gly Leu Ser Glu Val Thr Cys Thr Asp Asp
            820                 825                 830

Val Ser Met Pro Lys Leu Lys Lys Leu Leu Leu Thr Gly Phe His Cys
        835                 840                 845

Gly Ile Ser Leu Ser Glu Arg Leu Lys Lys Leu Ser Lys
    850                 855                 860

<210> 4
<211> 2586
<212> DNA
<213> Lycopersicon esculentum

<400> 4
atggctgaaa ttcttcttac atcagtaatc aataaatctg tagaaatagc tggaaattta 60
ctgattcaag aaggaaagcg tttatattgg ttgaaagagg atatcgattg gctccagaga 120
gaaatgagac acattcgatc ttatgttgac aacgcaaagg ccaaggaagc tggaggtgat 180
tcaagggtca aaaacttatt gaaagatatt caagaattgg caggtgatgt ggaggatctc 240
ttagatgact tccttccaaa aattcaacga tccaataagt tcaattattg ccttaagacg 300
agttcttttg cggatgagtt tgctatggag attgagaaga taaagagaag ggttgttgac 360
attgaccgaa taaggaaaac ttacaacatc atagatacag ataacaataa tgatgattgt 420
gttttgctgg atcggagaag attattccta catgctgatg aaacagagat catcggtttg 480
gatgatgact tcaatatgct acaagccaaa ttactcaatc aagatttgca ttatggagtt 540
gtttccatag ttggcatgcc cggtctgggg aaaacaactc ttgccaagaa actttatagg 600
ctcattcgtg atcaatttga gtgttctgga ctggtctacg tttcacaaca gccaagagcg 660
ggtgaaatct tacttgacat tgccaaacaa attggactga cggaacagaa aattaaggaa 720
aatttggagg acaacctgcg atcactcttg aaaataaaaa ggtatgttat cctcctagat 780
gacatttggg atgttgaaat ttgggatgat ctgaaacttg tccttcctga atgtgactca 840
aaagtcggca gtagaatgat aatcacgtct cgaaatagta atgtaggcag atacatagga 900
ggggaatcct ccctccatgc attgcaaccc ctagaatccg agaaaagctt tgaactcttt 960
accaagaaaa tctttaattt tgatgataat aatagttggg ccaatgcttc acctgacttg 1020
gtgaatattg gtagaaatat agctgggaga tgtggaggta taccgctagc catagtggtg 1080
actgcaggca tgttaagggc aagagaaaga acagaacatg cgtggaacag agtacttgag 1140
agtatgggcc ataaagttca agatggatgt gctaaggtat tggctctcag ttacaatgat 1200
ttaccgattg cctcaaggcc atgtttcttg tactttagcc tttaccccga ggaccatgaa 1260
attcgtgctt ttgatttgat aaatatgtgg attgctgaga agtttattgt agtaaatagt 1320
ggtaataggc gagaggctga ggatttggcg gaggacgtcc taaatgattt ggtttctaga 1380
aacttgattc aacttgccaa aaggacatat aatggaagaa tttcaagttg tcgcatacat 1440
gacttgttac atagtttgtg tgtggacttg gctaaggaaa gtaacttctt tcacaccgcg 1500
catgatgtat ttggtgatcc cggcaatgtc gctaggcttc gaaggattac attctactct 1560
gacaatgtca tgattgagtt cttcggttct aatcctaagc ttgagaagct tcgtgtactt 1620
ttctgtttca caaaagaccc ttccatattt tctcatatgg cttgttttga cttcaaattg 1680
ttgcacacat tggttgtagt catgtctcaa agttttcaag catatgtcac tatcccaagc 1740
aaatttggga acatgacttg cttacgctat ctgaaattgg aggggaatat ttgtggaaaa 1800
ctgccaaata gtattgtcaa gctcacacgt ctagagacca tagacattga tcgacgtagc 1860
ctcattcaac ttccttctgg tgtttgggag tctaaacatt tgagacatct ttgttataga 1920
gattatggac aagcatgtaa cagttgcttt tctataagct cattttaccc aaacatttac 1980

tcattgcatc ctaacaatct acaaaccttg atgtggatac ctgataaatt ttttgaaccg 2040
aggttgttgc accgattgat caatttaaga aaactgggta tactgggagt gtccaattca 2100
accgttaaga tattatcaac atgtcgccct gtgccaaagg cgctaaaggt tctgaagctc 2160
aggtttttca gtgatccgag tgagcaaata aacttgtcat cctatccaaa aattgttaag 2220
ttgcatttga atgttgacag aacaatagcc ttgaactctg aagcattccc tccaaatatt 2280
atcaagctta ctcttgtctg ctttatggta gacagttgtc tactggcagt gcttaagaca 2340
ttacccaaat taagaaaact taaaatggtc atctgcaagt ataatgaaga aaagatggct 2400
ctctcgggcg aggcaaatgg ttatagcttt ccgcaacttg aagttttgca tattcatagc 2460
ccgaatgggt tgtctgaagt aacatgcacg gatgatgtca gtatgcccaa attgaaaaag 2520
ctgttactta caggattcca ttgcggaatc agtttatcgg aacggcttaa aaagctgagt 2580
aaatga 2586

<210> 5
<211> 264
<212> PRT
<213> Tomato mosaic virus

<400> 5
Met Ala Leu Val Val Lys Gly Lys Val Asn Ile Asn Glu Phe Ile Asp
1               5                   10                  15

Leu Ser Lys Ser Glu Lys Leu Leu Pro Ser Met Phe Thr Pro Val Lys
            20                  25                  30

Ser Val Met Val Ser Lys Val Asp Lys Ile Met Val His Glu Asn Glu
        35                  40                  45

Ser Leu Ser Glu Val Asn Leu Leu Lys Gly Val Lys Leu Ile Glu Gly
    50                  55                  60

Gly Tyr Val Cys Leu Val Gly Leu Val Val Ser Gly Glu Trp Asn Leu
65                  70                  75                  80

Pro Asp Asn Cys Arg Gly Gly Val Ser Val Cys Met Val Asp Lys Arg
                85                  90                  95

Met Glu Arg Ala Asp Glu Ala Thr Leu Gly Ser Tyr Tyr Thr Ala Ala
            100                 105                 110

Ala Lys Lys Arg Phe Gln Phe Lys Val Val Pro Asn Tyr Gly Ile Thr
        115                 120                 125

Thr Lys Asp Ala Glu Lys Asn Ile Trp Gln Val Leu Val Asn Ile Lys
    130                 135                 140

Asn Val Lys Met Ser Ala Gly Tyr Cys Pro Leu Ser Leu Glu Phe Val
145                 150                 155                 160

Ser Val Cys Ile Val Tyr Lys Asn Asn Ile Lys Leu Gly Leu Arg Glu
                165                 170                 175

Lys Val Thr Ser Val Asn Asp Gly Gly Pro Met Glu Leu Ser Glu Glu
            180                 185                 190

Val Val Asp Glu Phe Met Glu Asn Val Pro Met Ser Val Arg Leu Ala

195                 200                 205

Lys Phe Arg Thr Lys Ser Ser Lys Arg Gly Pro Lys Asn Asn Asn Asn
    210                 215                 220

Leu Gly Lys Gly Arg Ser Gly Gly Arg Pro Lys Pro Lys Ser Phe Asp
225                 230                 235                 240

Glu Val Glu Lys Glu Phe Asp Asn Leu Ile Glu Asp Glu Ala Glu Thr
                245                 250                 255

Ser Val Ala Asp Ser Asp Ser Tyr
            260

<210> 6
<211> 795
<212> DNA
<213> Tomato mosaic virus

<400> 6
atggctctag ttgttaaagg taaggtaaat attaatgagt ttatcgatct gtcaaagtct 60
gagaaacttc tcccgtcgat gttcacgcct gtaaagagtg ttatggtttc aaaggttgat 120
aagattatgg tccatgaaaa tgaatcattg tctgaagtaa atctcttaaa aggtgtaaaa 180
cttatagaag gtgggtatgt ttgcttagtc ggtcttgttg tgtccggtga gtggaattta 240
ccagataatt gccgtggtgg tgtgagtgtc tgcatggttg acaagagaat ggaaagagcg 300
gacgaagcca cactggggtc atattacact gctgctgcta aaaagcggtt tcagtttaaa 360
gtggtcccaa attacggtat tacaacaaag gatgcagaaa agaacatatg gcaggtctta 420
gtaaatatta aaaatgtaaa aatgagtgcg ggctactgcc ctttgtcatt agaatttgtg 480
tctgtgtgta ttgtttataa aaataatata aaattgggtt tgagggagaa agtaacgagt 540
gtgaacgatg gaggacccat ggaactttcg gaagaagttg ttgatgagtt catggagaat 600
gttccaatgt cggttagact cgcaaagttt cgaaccaaat cctcaaaaag aggtccgaaa 660
aataataata atttaggtaa ggggcgttca ggcggaaggc ctaaaccaaa aagttttgat 720
gaagttgaaa aagagtttga taatttgatt gaagatgaag ccgagacgtc ggtcgcggat 780
tctgattcgt attaa 795

<210> 7
<211> 20
<212> DNA
<213> Artificial Sequence

<220>
<223> Description of Artificial Sequence:
      oligonucleotide

<400> 7
gaactcaccg cgacgtctgt 20

<210> 8
<211> 21
<212> DNA
<213> Artificial Sequence

<220>
<223> Description of Artificial Sequence:
      oligonucleotide

<400> 8
gtcggcatct actctattcc t                                                21


<210> 9
<211> 21
<212> DNA
<213> Artificial Sequence

<220>
<223> Description of Artificial Sequence:
      oligonucleotide

<400> 9
cgtcctgtag aaaccccaac c                                                21


<210> 10
<211> 21
<212> DNA
<213> Artificial Sequence

<220>
<223> Description of Artificial Sequence:
      oligonucleotide

<400> 10
cggcgtggtg tagagcatta c                                                21


<210> 11
<211> 19
<212> DNA
<213> Artificial Sequence

<220>
<223> Description of Artificial Sequence:
      oligonucleotide

<400> 11
agctggctgg actttcctt                                                   19


<210> 12
<211> 20
<212> DNA
<213> Artificial Sequence

<220>
<223> Description of Artificial Sequence:
      oligonucleotide

<400> 12
cagcatggct tgagtctttg                                                    20

<210> 13
<211> 28
<212> DNA
<213> Artificial Sequence

<220>
<223> Description of Artificial Sequence:
      oligonucleotide

<400> 13
cttgacaaga ctgcagcgag tgattgtc                                           28

<210> 14
<211> 28
<212> DNA
<213> Artificial Sequence

<220>
<223> Description of Artificial Sequence:
      oligonucleotide

<400> 14
ctactacact cacgttgctg tgatgcac                                           28

<210> 15
<211> 46
<212> DNA
<213> Artificial Sequence

<220>
<223> Description of Artificial Sequence:
      oligonucleotide

<400> 15
ttttccatgg ctgaaattct tcttacatca gtaatcaata aatctg                       46

<210> 16
<211> 40
<212> DNA
<213> Artificial Sequence

<220>
<223> Description of Artificial Sequence:
      oligonucleotide

<400> 16
ctgacctgcc atggtgttca tttactcagc tttttaagcc                             40

<210> 17
<211> 137
<212> DNA
<213> Artificial Sequence

<220>
<223> Description of Artificial Sequence: AGLINK
      multicloning site

<400> 17
gcggccgctc cggattcgaa ttaattaacg tacgaagctt gcatgcctgc agtgatcacc 60
atggtcgact ctagaggatc cccgggtacc gagctcgaat tcggcgcgcc caattgattt 120
aaatggccgc tgcggcc 137